Europäisches Patentamt

European Patent Office

Office européen des brevets

(11) Publication number: **0 098 688**
**A1**

(12) **EUROPEAN PATENT APPLICATION**

(21) Application number: 83303169.3

(22) Date of filing: 02.06.83

(51) Int. Cl.³: **A 61 M 25/00**

(30) Priority: 03.06.82 US 384570

(43) Date of publication of application: 18.01.84
Bulletin 84/3

(84) Designated Contracting States: BE DE FR GB SE

(71) Applicant: **Mullaney, Brian A., 540 Coldstream Drive,
Berwyn Pennsylvania 19312 (US)**

(72) Inventor: **Mullaney, Brian A., 540 Coldstream Drive,
Berwyn Pennsylvania 19312 (US)**

(74) Representative: **Dealtry, Brian, Eric Potter &
Clarkson 14, Oxford Street, Nottingham NG1 5BP (GB)**

(54) **Intratubular medication dispenser.**

(57)    An intratubular medication dispenser (10) has an outer tube
(14) and an inner tube (12) for insertion into the body passageway
(26) of a patient. The inner tube (12) is used for injecting a
medicinal fluid into a body cavity of withdrawing bodily secretions
or a medicinal fluid from a body cavity; the outer tube (14), by way
of a plurality of perforations and the annular space formed
between the inner and outer tubes (12, 14), is used for dispensing
medicinal fluid at preselected locations along the length of the
tube (14) in the body passageway (26).

## INTRATUBULAR MEDICATION DISPENSER

### Field of the Invention

This invention relates generally to surgical instruments and accessories, and more specifically to an intubation device for dispensing a medicinal fluid at one or more points along the length of the device, and simultaneously conducting another function, such as removing bodily secretions from or injecting another medicinal fluid to a point at the distal end of the device.

As used herein, the term "medicinal fluid" includes anesthetic agents, antibiotics, analgesics, antimicrobial agents, antibacterial agents, antiinflammatory agents, chemotherapeutic agents, saline solution, and food compositions, or any other composition, pharmaceutical, or otherwise that may be used for a medicinal or life-supporting purpose. The "function" conducted at the distal end of the device may also include essentially any surgical, observation, or monitoring procedure, which requires an elongated connecting link, normally in the form of a tube and generally referred to herein as a tube, to a point outside the patient's body.

### Description of the Prior Art

Various intubation devices have been developed for withdrawing fluid from a patient's body cavity or injecting fluid into the body. Examples of these types of devices are catheters and infusion cannula tubes. Sometimes, these devices must remain within the patient for extended periods of time. When such devices remain several days in the stomach or duodenum or some other body cavity or passageway, extreme discomfort and pain may be experienced by the patient. Irritation of the tissue with which the outer surface of the tube remains in contact is generally responsible for this pain or discomfort. For example, in nasogastric intubation, fluids may be injected into or withdrawn from the stomach cavity, while the nasal and upper

0098688

-3-

throat passageways are the areas in which pain is experienced by the patient.

In order to relieve the pain or discomfort, an anesthetic agent or some other medication may be introduced into the body passageway. The introduction of the anesthetic agent, however, leaves much to be desired, since no way is known to introduce the anesthetic agent concurrent with the main intubation function. Moreover, localized application of the medication to the irritated area is not provided in known prior art methods and devices.

Examples of prior art devices which may be useful for the administration of medicinal fluids, and which may be considered related to the subject matter of the present invention, are disclosed in the following U.S. patents: 4,280,500; 3,885,651; and 4,182,326. All these references are directed to an intubation device consisting of a flexible tube having a plurality of openings in the walls of the tube for insertion of medication. None of the cited references, however, may be used for the administration of one medicinal fluid to one body location and the administration of another medicinal fluid to another body location. Moreover, none of the cited references may be used for the withdrawal of bodily secretions from one location and the

0098688

-4-

simultaneous application of a medication to a second location.

It is, therefore, an object of this invention to provide a device for intubation of a patient over an extended period of time in order to effect some medical function, such as to inject or withdraw medicinal fluid or bodily secretion into or from a particular location in a body cavity or passageway while, at the same time, avoiding or obviating the pain or discomfort that might otherwise be caused by the device.

## Summary of the Invention

An intubation device has an outer tube for insertion into the body passageway of a patient, and an inner tube which has a length greater than that of the outer tube. Normally, both tubes are flexible. The inner tube, which is disposed within the outer tube to form an annular, crescent-shaped or some other closed cross-sectional shaped space therebetween (sometimes referred to hereafter as an annular space), has a distal end for conducting some function, such as injecting or withdrawing fluid from the patient. A plurality of perforations are formed through the outer tube which are in communication with the annular space for dispensing medicinal fluid to the body passageway at one or more points along the length of the intubation device.

-5-

Brief Description of the Drawings

Fig. 1 shows the use of the intratubular medication dispenser of the present invention in a nasogastric intubation of a patient.

Fig. 2 is a view, partially in section, of the intratubular medication dispenser shown in Fig. 1.

Fig. 3 is a cross-sectional view taken along line 3-3 of Fig. 2.

Fig. 4 is a view of another embodiment of the intratubular medication dispenser of the present invention.

Fig. 5 is a cross-sectional view taken along line 5-5 of Fig. 4.

Description of the Preferred Embodiment

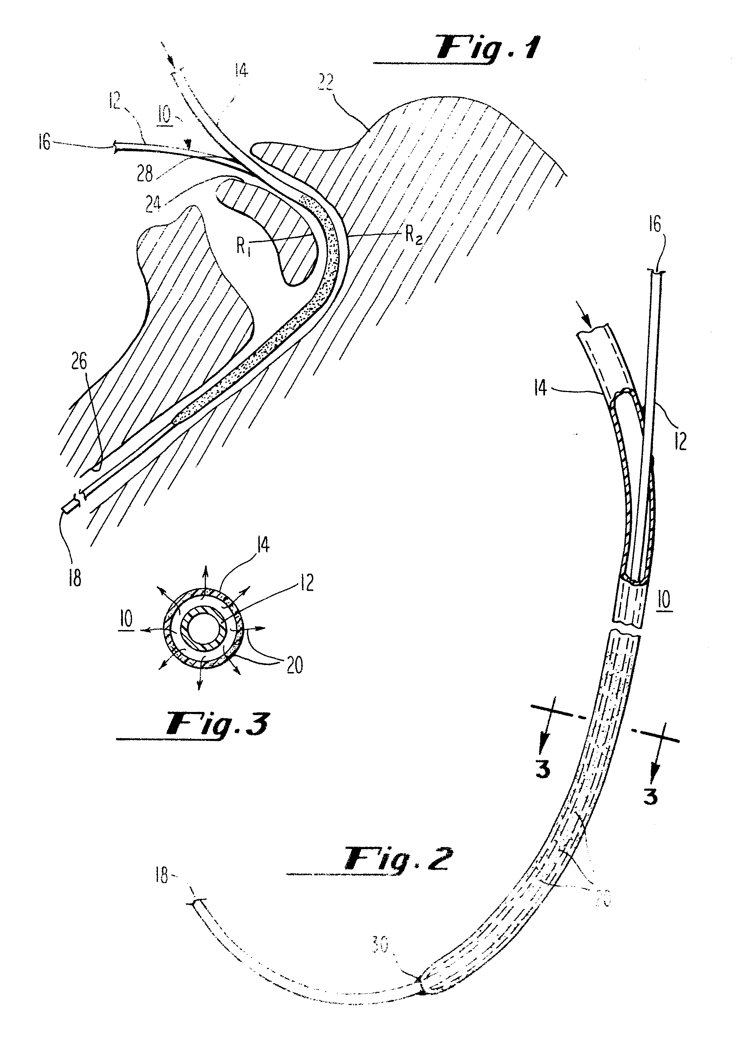

Referring to Figs. 1, 2, and 3, the intratubular medication dispenser 10 of the present invention (in the embodiment shown in Figs. 1-3) has an inner tube 12 and an outer tube 14, one or both of which may be flexible to facilitate insertion and use. The inner tube or cannula is adapted to be disposed within outer tube 14 in telescopic and mating relation. In this connection,

-6-

inner tube 12 may be considered as a catheter for providing fluid communication between proximal end 16 and distal end 18. Tube 12, in the nasogastric application shown in Fig. 1, may be used for injecting or withdrawing fluid from a patient's stomach, in which distal end 18 would be located. In other applications, distal end 18 may be located in a blood vessel or the urinary tract or in some other part of the patient's digestive tract. Proximal end 16 normally would be located externally of the patient's body.

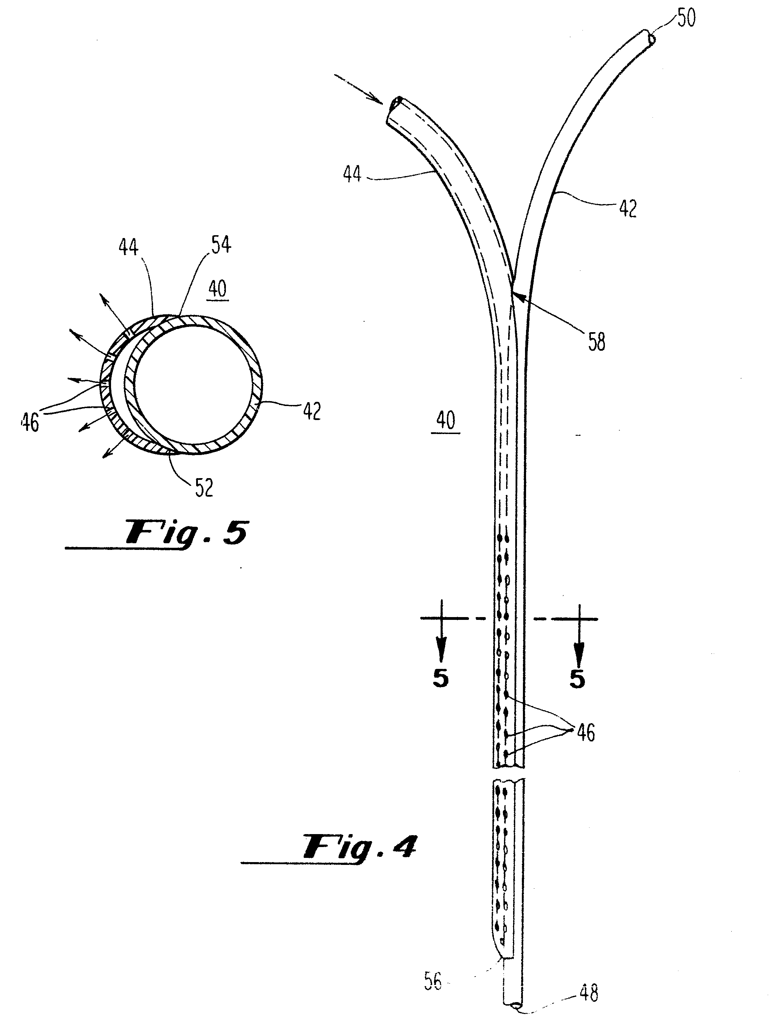

Outer tube 14 may entirely surround inner tube 12 along a portion of the longitudinal dimension of inner tube 12, as shown in Figs. 2 or 3, or it may surround only a portion of tube 12, as shown in Figs. 4 and 5. Outer tube 14 is provided with one or more perforations 20. The number of perforations may vary but are suitably arranged radially about the outer tube, as shown in Fig. 3, to provide localized medicinal fluid dispensing outlets. In this manner, a means is provided for dispensing medicinal fluid from the annular space between the two tubes, through the perforations in the outer tube, and into the passageway.

-7-

By way of example, Fig. 1 shows intratubular medication dispenser 10 in operation while inserted into nasogastric passageway 26 of patient 22 by way of nasal cavity 24. Distal end 18 of dispenser 10 continues down the passageway into the stomach (not shown) or duodenum (not shown) of patient 22. Moving in a direction away from distal end 18, inner tube 12 passes into outer tube 14 forming an annular space therebetween. Inner tube 12 continues extending longitudinally in a telescopic relationship within outer tube 14 for a portion thereof. Moving in a direction toward proximal end 16, inner tube 12 exits from the outer tube by way of a sealed opening 28, the latter formed through the wall of outer tube 14, as shown in Fig. 2. It will be understood that opening 28 may be of any reasonable size to easily permit inside tube 12 to pass out of outside tube 14. In addition, opening 28 may preferably be located near the proximal end at a location external to patient 22. At the proximal end, inner tube 12 may be connected, in a conventional manner, to a vial or syringe (not shown) for either injecting or withdrawing fluid from distal end 18. Similarly, outer tube 14 may be connected, also in a conventional manner, to a vial or syringe (not shown) for injecting medicinal fluid into the body passageway by way of the matrix of perforations 20.

-8-

In its fully inserted position, intratubular medication dispenser 10 gently curves to follow the nasogastric contours of patient 22. By forming both tubes of a soft plastic material, injury to the body passageway may be prevented during intubation of patient 22.

Typically, tubes 12 and 14 may be molded of flexible plastic material, such as plasticized vinyl copolymer resin, PVC, polyethylene, polyfluorocarbon (such as Teflon) or any other well-known thermoplastic resins or other molding materials which are resistant to attack by mucous or other glandular secretions of the body and by the medicinal fluid to be transmitted.

In one embodiment, the internal diameter of outer tube 14 is just slightly larger than the external diameter of inner tube 12, and tubes 12 and 14 are otherwise adapted so that inner tube 12 may be slidably insertable into outer tube 14. Alternatively, tubes 12 and 14 may be molded together or bonded at the points where inner tube 12 exits tube 14.

The diameters and shapes of tubes 12 and 14 may vary, of course, depending upon patient body size and other characteristics and the location of the intubation in the body.

0098688

-9-

By way of example, for nasogastric intubation with normal adult patients, the external diameter of outer tube 14 may typically be 5.0 mm and the external diameter of inner tube 12 may be 3.5 mm.

In another specific embodiment of the invention, in order to laterally position outer tube 14 over inner tube 12 and ensure that one tube does not move longitudinally with respect to the other tube, outer tube 14 is tapered to a smaller diameter in a direction toward distal end 18. Tapered portion 30, shown in Fig. 2, is a smooth and uniform taper, beginning at a diameter size equal to the diameter of outer tube 14 and tapering to the diameter of inner tube 12. To ensure additional rigidity, cement may be applied between tapered portion 30 and inner tube 12. The cement also seals the annular space between the two tubes, thereby preventing any fluid injected into outer tube 14 from dripping down beyond tapered portion 30. Of course, the sealing method may be any other method known or developed by the art, such as heat fusion.

As shown in Figs. 2 and 3, a plurality of perforations 20 are situated immediately above tapered portion 30 about outer tube 14. In this embodiment, perforations 20 are evenly distributed circumferentially and longitudinally, thereby uniformly communicating with the annular space between both tubes, thus providing a 360° dispensing pattern into the body passageway. The fineness of the spray or mist or the rate of flow delivered by perforations 20 will, of course, be dependent upon the hole size of perforations 20. The smaller the hole size, the finer will be the spray or the slower will be the rate of flow of fluid delivered. In this manner, the rate of medicinal fluid delivery may be controlled depending upon the type of intubation.

In still another embodiment, a multiplicity of perforations may be provided by utilizing a mesh or porous material of construction for all or a part of outer tube 14. The term "perforation" as used herein may otherwise refer to slits or essentially any other form of opening, as well.

It will be understood that the location of the dispensing pattern delivered to the patient may be controlled by positioning the matrix of perforations 20 at an appropriate location along inner tube 12. Such location will vary, of course, depending on the type of intubation. For example, in nasogastric application, the matrix of perforations may be positioned to lie along the inside radius $R_1$ and the outside radius $R_2$ of the curvature of the nasal passageway of patient 22 and may be approximately 30 cm long. In this manner,

-11-

outer tube 14 may deliver anesthetic fluid, for example, to the patient to relieve the main area of discomfort experienced in the nose and upper throat during nasogastric intubation.

A second embodiment of the invention is shown in Figs. 4 and 5. There shown is intratubular medication dispenser 40 consisting of tubes 42 and 44. Tube 42 may be used for fluid communication between distal end 48 and proximal end 50; as previously described, tube 42 may be used for injecting or withdrawing fluid from distal end 48 resident within a patient's body. Tube 44 having a matrix of perforations 46 may be used, as described earlier, for delivering medicinal fluids to the patient's body passageway for minimizing discomfort during intubation or effecting other medical purposes.

In order to couple tube 42 with tube 44, an elongated, longitudinal slit 58 is made in tube 44. Slit 58 is made sufficiently large, leaving approximately one-half the circumference of tube 44 intact. End walls 52 and 54 formed by the longitudinal slit are then abutted against the outer wall of tube 42, as shown in Fig. 5. In this manner, only about one-half of tube 42 is encompassed or surrounded by tube 44, in the construction shown in Fig. 5. Essentially, any part of the outside curvature of the inner tube could be encompassed in other constructions.

-12-

Still further, the inner and outer tubes may be formed together to make a single simple outer shape (circular, for example) internally divided to form the inner and outer tubes by a partition of the circular cross-sectional area. The two "tubes" in such a design would in effect be parallel passageways within a closed outer shape.

Referring again to the construction shown in Figs. 4 and 5, to permanently affix end walls 52 and 54 onto the outer wall of tube 42, cement may be applied, or some other method may be used such as heat fusion. It will also be noted that tube 44 may have a tapered portion 56 which tapers in a direction toward distal end 48, ending in a diameter size equal to the diameter of tube 42. As described earlier, tapered portion 56 may also be permanently affixed onto the outer wall of tube 42 with cement or another type of sealant.

With cement applied at end walls 52 and 54 and at tapered portion 56, a sealed arch-like or crescent-shaped space is formed between tube 42 and tube 44. Since a matrix of perforations 46 is provided through the wall of tube 44 which is in communication with the arch-like space, any fluid originating from the proximal end of tube 44 may be dispensed traversely out of said space.

-13-

As shown in Figs. 4 and 5, perforations 46 are evenly distributed circumferentially and longitudinally about the arch-like portion of tube 44. As described above with respect to the embodiment of Figs. 1-3, perforations 46 may be disposed in essentially any pattern and at essentially any preselected location in tube 44.

It will be understood that the advantages of the embodiment shown in Fig. 4 is that two separate and distinct fluid communication channels are provided by way of tubes 42 and 44, respectively, and that the dispensing of fluid from tube 44, by way of perforations 46, may be directionalized in a preferred direction, instead of being dispensed omnidirectionally. In addition, the overall diameter of the device may be reduced while still providing an intubation means with means for concurrently dispensing medicinal fluid in a selectively localized sidewall area of the device. This embodiment is considered particularly adapted for the relief of discomfort and pain in nasogastric intubation in which the area of irritation is highly localized and well defined.

Other constructions of such partially surrounding outer tube designs are likely to be equally useful.

In this regard, while the foregoing description has been drawn to two specific embodiments of the inven-

-14-

tion, it will be understood by those skilled in the art that changes in form and detail are encompassed within the scope and spirit of the invention, and the appended claims are intended to be construed to include such modified forms of the invention.

-15-

Claims:

1. An intubation device comprising an outer tube for insertion into a body passageway; an inner tube having a length greater than that of said outer tube and surrounded partially or completely by said outer tube to form an annular space therebetween, said inner tube having a distal end for injecting or withdrawing fluid from said user; said outer tube having a plurality of perforations formed through said outer tube in communication with said annular space for dispensing medicinal fluid to said body passageway.

2. The intubation device of Claim 1, wherein said outer tube has a constant diameter for a substantial length of said outer tube and then uniformly tapers in a direction toward said distal end into a diameter size of said inner tube, thereby longitudinally fixing said outer tube in relation to said inner tube.

-16-

3.   The intubation device of Claim 1, wherein said perforations are located circumferentially and longitudinally along said outer tube to form an omnidirectional and uniform dispersion pattern of medicinal fluid.

4.   The intubation device of Claim 1, wherein said outer tube has an opening for permitting the insertion of said inner tube, and said plurality of perforations are located between said opening and said distal end.

5.   The intubation device of Claim 1, wherein said inner tube and said outer tube contain fluids, said fluid in said inner tube being separate and distinct from said fluid contained in said outer tube.

6.   An intubation device comprising:
a first tube for insertion into the body passageway of a patient, said first tube having an elongated longitudinal slit forming lateral end walls; a second tube having a length greater than that of said first tube and a distal end for injecting or withdrawing fluid from said user;

-17-

said second tube bonded to said end walls forming an enclosed space between said first tube and said second tube;

a plurality of perforations formed through said first tube, said perforations located between said end walls and in fluid communication with said space;

a first tube being adapted to dispense medicinal fluid onto said body passageway by way of said perforations.


7. An intubation device comprising a first tube having a proximal end, adapted to be positioned outside a patient's body, and a distal end adapted to be positioned inside a patient's body, and a longitudinal section of a second tube adjacent said first tube, said second tube having a first terminus toward said distal end of said first tube, said second tube together with said first tube defining an enclosed space between said tubes, said second tube including at least one opening at a preselected location along the length thereof, through which said enclosed space is in communication with the space surrounding said second tube, said second tube further including a tubular extension thereof in communication with said enclosed space, said tubular extension also adapted to be positioned outside a patient's body.

0098688

-18-

8. An intubation device, as recited in Claim 7, wherein said second tube partially surrounds said first tube along a part of the length thereof.

9. An intubation device, as recited in Claim 7, wherein said second tube surrounds said first tube along a part of the length thereof.

10. An intubation device, as recited in Claim 3, wherein said second tube includes a plurality of openings disposed at preselected locations along the length thereof.

11. An intubation device, as recited in any one of Claims 7, 8, 9, or 10 wherein said second tube comprises, along at least a part of its length, a porous material providing a multiplicity of openings through which said enclosed space is in communication with said surrounding space.

_Fig.1_

_Fig.3_

_Fig.2_

0098688

_Fig. 5_

_Fig. 4_

## DOCUMENTS CONSIDERED TO BE RELEVANT

EP 83303169.3

| Category | Citation of document with indication, where appropriate, of relevant passages | Relevant to claim | CLASSIFICATION OF THE APPLICATION (Int. Cl. 3) |
|---|---|---|---|
| X,Y | <u>GB - A - 2 017 499</u> (DR. E. FRESE-NIUS) <br> * Fig. 3; page 2, lines 2-16; claims 1,2 * | 1-5,10 11 | A 61 M 25/00 |
| D,Y | <u>US - A - 4 280 500</u> (K. ONO) <br> * Fig. 1,2; abstract * | 1-5,10 11 | |
| X | <u>US - A - 4 037 599</u> (J.D. RAULERSON) <br> * Fig. 1,2,4,10; column 5, line 37 - column 6, line 8 * | 1-5,11 | |
| X <br> A | <u>DE - B - 2 024 791</u> (D. KANIASTAS) <br> * Fig. 5; column 4, lines 37-53 * | 1-3,5, 4,7,9 | |
| A | <u>US - A - 4 193 406</u> (W.J. JINOTTI) <br> * Fig. 5; column 3, lines 21-56 * | 7 | **TECHNICAL FIELDS SEARCHED (Int. Cl. 3)** <br><br> A 61 M 1/00 <br> A 61 M 5/00 <br> A 61 M 25/00 <br> A 61 M 31/00 |
| A | <u>US - A - 3 853 130</u> (D.S. SHERIDAN) <br> * Fig. 6,7 * | 6-9 | |

The present search report has been drawn up for all claims

| Place of search | Date of completion of the search | Examiner |
|---|---|---|
| VIENNA | 12-10-1983 | LUDWIG |